Europäisches Patentamt

(19) European Patent Office

Office européen des brevets

(11) **EP 0 787 500 B1**

(12) **EUROPEAN PATENT SPECIFICATION**

(45) Date of publication and mention
of the grant of the patent:
**01.12.1999 Bulletin 1999/48**

(51) Int. Cl.$^6$: **A61M 1/34**, A61M 1/36

(21) Application number: **96830049.1**

(22) Date of filing: **06.02.1996**

(54) **Device for extracorporeal removal of toxins, in particular cytokines**

Vorrichtung zum extrakoporealen Entfernen von Toxinen, insbesondere Zytokinen

Dispositif de seperation de toxines en particulier de cytokines

(84) Designated Contracting States:
**DE ES FR GR IT NL**

(43) Date of publication of application:
**06.08.1997 Bulletin 1997/32**

(60) Divisional application:
**99109637.1 / 0 958 839**

(73) Proprietor: **BELLCO S.p.A.**
**I-41037 Mirandola (IT)**

(72) Inventors:
• **Wratten Mary Lou**
**41036 Medolla (IT)**
• **Tetta, Ciro**
**41037 Mirandola (IT)**

(74) Representative:
**Plebani, Rinaldo et al**
**STUDIO TORTA S.r.l.,**
**Via Viotti, 9**
**10121 Torino (IT)**

(56) References cited:
**EP-A- 0 451 429       WO-A-90/12632**
**FR-A- 2 390 965**

• **DATABASE WPI Section Ch, Week 9504 Derwent**
**Publications Ltd., London, GB; Class A96, AN**
**95-027288 XP002006819 & JP 06 312 017 A**
**(ASAHI MEDICAL CO LTD) , 8 November 1994**

Remarks:
The file contains technical information submitted
after the application was filed and not included in
this specification

## Description

The present invention relates to a device for purifying blood which provides for a highly effective method of purifying blood in patients affected with acute organ failure, and which may be used without difficulty in conjunction with other known blood purifying methods. Though preferably applied to the treatment of acute organ failure, possibly in conjunction with or caused by septic shock, the device may also be used to advantage in improving the effectiveness of known methods of treating patients affected with chronic organ failure.

In patients suffering from organ failure, the blood gradually retains endless quantities of various substances commonly referred to as "toxins", and which, in healthy subjects, the relative organs are normally capable of eliminating from the bloodstream. The accumulation of such toxins, if not treated, results in a wide range of organic malfunctions and eventually in death; and acute syndromes are characterized by a high percentage of cytokines among the toxins for removal.

The most commonly used systems for purifying the blood of toxins are by adsorbing them on solid media (hemo- and plasmaperfusion), or by ultrafiltering the blood or plasma through appropriate semipermeable membranes, either by convection with the aid of a pressure gradient (TMP) through the membrane (hemo- or plasmafiltration), or by diffusion by bringing the blood or plasma to be purified into contact with one side of the membrane, and an appropriately formulated wash solution into contact with the opposite side (hemodialysis).

All the above systems, however, present drawbacks. Hemoperfusion consists in percolating the blood directly through a filter of adsorbent material, which must therefore be made highly biocompatible. This is usually achieved by covering the adsorbent particles with appropriate material, which, however, seriously impairs the toxin-retaining capacity of the particles. In the case of plasmaperfusion, the blood is first filtered to separate the plasma, which is then percolated through the adsorbent material. Though this to some extent solves the problem of biocompatibility during perfusion, the increase in the viscosity of the blood during filtration may result in extensive clotting through the membrane, so that in any case the blood must be treated with anticoagulants (heparin).

Hemo- and plasmafiltration, on the other hand, only provide for removing high-molecular-weight toxins, and produce a considerable weight loss which must be compensated by feeding an infusion solution into the patient's blood. According to EP-A-0 451 429 the above problem may be partly solved by regenerating the ultrafiltrate, by absorbing the medium-high-molecular-weight toxins in it by percolating it through uncoated-activated-carbon-based hemoperfusion cartridges such as DETOXIL 2™ produced by SORIN BIOMEDICA of Saluggia, Italy, so that the regenerated ultrafiltrate may be used, as it is or with additions, as an infusion solution.

Hemodialysis, particularly if combined with one or more of the above methods, is the most effective, but is relatively lengthy in terms of purification time, and therefore fails to provide for sufficient toxin removal in acute crises. In particular, cytokine removal is fairly poor, so that, at present, organic malfunctions caused by acute organ failure can be no more than delayed as opposed to fully prevented.

It is an object of the present invention to overcome the aforementioned drawbacks by enabling blood to be purified rapidly and effectively of any and all toxins, including cytokines.

According to the present invention, there is provided a device according to Claim 1.

The ultrafiltrate or plasmafiltrate is subjected to a double adsorption step, first through a column of activated carbon, and then through a column packed with hydrophobic or ion-exchange resin.

In particular, the resin used is a reticulate aromatic polymer, macroreticulate and/or macroporous, or synthetic carbonaceous resin such as AMBERLITE™, AMBERCHROM™, AMBERSORB™.

The present invention therefore relates to an adsorbent filter in conjunction with an ultrafilter or plasmafilter to enable rapid, highly effective removal of cytokines from a stream of blood; wherein use is made of an ultrafilter or plasmafilter permitting the majority of the cytokines present in the stream of blood to pass into a stream of ultrafiltrate or plasmafiltrate derived by convection from the stream of blood by means of the ultrafilter or plasmafilter; and wherein the ultrafiltrate or plasmafiltrate is fed through an adsorbent filter wherein the adsorbent material comprises uncoated activated carbon, hydrophobic or ion-exchange polystyrene resin, or mixtures thereof.

A number of non-limiting embodiments of the present invention will be described by way of example with reference to the accompanying drawings, in which:

Figure 1 shows, schematically, a test circuit implementing a device in accordance with the present invention, and used for testing the device according to the invention;
Figures 2 to 7 show test graphs illustrating removal of the principal cytokines in the ultrafiltrate using the Figure 1 circuit.

Roughly speaking, the present invention is based on the observation that uncoated activated carbon - which, according to EP-A-0 451 429, has proved a valuable aid in purifying an ultrafiltrate, by adsorption, of toxic substances in general (and $\beta 2$microglobulin in particular) of a molecular weight ranging in particular from 300 to 1,500 Dalton (theoretically the adsorption spectrum of activated carbon may range from 100 to 20,000 Dalton) and present in abnormal quantities in patients affected with chronic uremia - may surprisingly also be used - particularly in conjunction with hydrophobic and/or ion-exchange resins - for treating acute patients, to also enable rapid, practically total removal of the class of toxins known as cytokines, and which at present are not effectively removed by any known method or device.

In view of the notoriously adsorbent nature of activated carbon within the above range of molecular weights, it may be considered obvious that at least part of the cytokines would be retained by an activated-carbon filter, the molecular weight of the principal known cytokines ranging between 9,000 and 17,000 Dalton (only IL-6 has a molecular weight of 26,000 Dalton, i.e. well outside the known adsorption range of activated carbon). To achieve the objects of the present invention, however, as opposed to merely capturing at least part of the cytokines present in the bloodstream of acute patients, it is of vital importance to eliminate them as rapidly as possible.

Up to now, no indication has been given in existing literature that activated carbon - especially if used, as will be seen, in conjunction with resins - is also capable of ensuring sufficiently rapid removal of cytokines to permit effective treatment of acute patients. Thanks to the insight of the Applicant's researchers, however, this capacity has now been brought to light, and has been optimized by treating the bloodstream of acute patients according to a specific sequence. To begin with, ultrafiltration or plasmafiltration is performed, whereby the stream of blood to be purified is brought into contact with a first side of a known semipermeable membrane, e.g. a polysulfone membrane (PSF, SPAN), through which a pressure gradient (TMP) is maintained to collect by convection, on a second side of the membrane opposite the first, a stream of ultrafiltrate or plasmafiltrate, which is shunted to a secondary circuit, while the rest of the stream of blood (eluate) is removed.

Using the device according to the present invention, the above step is performed using a membrane of such physical-chemical and permeability characteristics (porosity, hydrophobicity, etc.) as to permit most (substantially all) of the cytokines present in the incoming blood stream to pass through the membrane into the ultrafiltrate/plasmafiltrate stream. The ultrafiltrate/plasmafiltrate stream containing the cytokines to be eliminated is then fed by said secondary circuit through filtering means forming part of the secondary circuit and so located that the stream containing the cytokines to be eliminated is brought into contact with activated-carbon granules, e.g. inside a first filter cartridge (adsorption column), as well as with granules of a hydrophobic or ion-exchange resin mixed with the carbon granules or, preferably, inside a second filter cartridge (adsorption column) hydraulically in series with the first.

This provides not only for rapidly eliminating the cytokines, which are retained in the two cartridges, thus achieving the object of the present invention, but also, as described in EP-A-0 451 429, for regenerating the ultrafiltrate/plasmafiltrate (activated carbon is also capable of retaining any other medium-molecular-weight toxins such as hippuric acid, uric acid, $\beta$2microglobulin, etc.) which may thus be used as a reinfusion solution and added to the eluate from the ultrafilter/plasmafilter to form a stream of purified blood which is then fed back in known manner into the patient's body.

The cytokines are removed more rapidly and effectively (and, above all, the life of the filter cartridges of the secondary circuit is extended) if the activated carbon is also used in conjunction with a hydrophobic resin, e.g. resins with a reticulate aromatic polymer base, macroreticulate and/or macroporous resins, such as those known commercially as AMBERCHROM™ (styrene-methacrylate resin) and AMBERLITE™ (copolymer divinylbenzene-polystyrene resin), and/or a synthetic carbonaceous resin such as AMBERSORB™. All these resins, which are produced and marketed by ROHM&HAAS and normally used in the manufacture of fruit juices and other food products, conform with and are classified nontoxic by American FDA Standards nr 21 CFR 173.65, and may therefore also be employed for medical purposes. Nevertheless, it still remained to be discovered that such resins (which are normally used as fillers for chromatography columns or as immobilizing supports for active substances) were also effective - and moreover, as they are - in retaining cytokines, and, what is more, at such a rate as to enable them to be used in the treatment of acute organ failure syndromes.

The resins used in the present invention have a granule size of 20 to 100 (250-350 for AMBERLITE™) micron (thousandths of a millimeter), and a porosity of 100 to 300 Angstrom for AMBERCHROM™, 30 to 480 Angstrom for AMBERSORB™, and 130 to 150 Angstrom for AMBERLITE™.

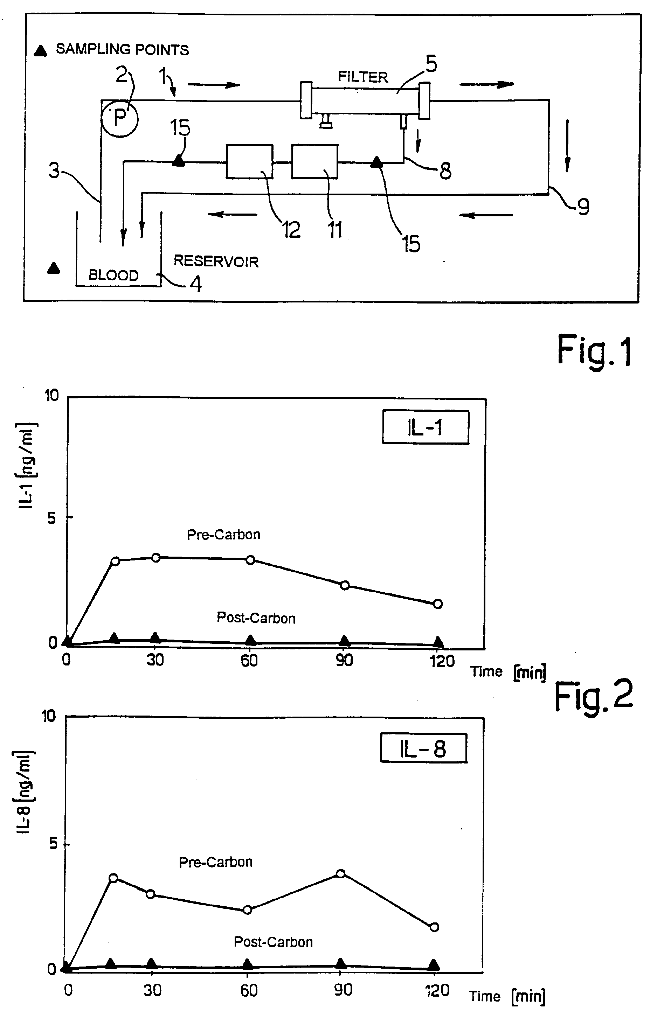

With reference to Figure 1, the device according to the invention has been tested in the circuit shown, and may be implemented in the device described in EP-A-0 451 429 by simply replacing the single uncoated-activated-carbon cartridge (DETOXIL-2™ by SORIN Biomedica of Saluggia) with two separate hemoperfusion cartridges hydraulically in series with each other and respectively packed with activated carbon and with one (or a mixture) of the above resins. The main components of such a device are already shown in the Figure 1 circuit, which comprises a main hydraulic circuit 1 presenting a circulating pump 2 and means for supplying a stream of blood to be purified, and which, in the example shown, are defined by a branch tube 3 immersed inside a reservoir 4 filled with a predetermined quantity of blood for treatment. Circuit 1 is connected hydraulically in series with a filter (ultrafilter or plasmafilter) 5 presenting a filtering means defined by a semipermeable membrane, and which provides for filtering the stream of blood by convection to form a stream of ultrafiltrate/plasmafiltrate inside a secondary circuit 8 branching hydraulically from circuit 1. The ultrafiltrate/plasmafiltrate flows along circuit 8 and is combined with a stream of eluate, issuing from filter 5 along a discharge tube 9, to form a stream of purified blood, which may be fed back using known means into the patient's body. For test purposes, in the example shown, circuit 8 and tube 9 both discharge into reservoir 4, so that the blood in the reservoir is batch treated cyclically for a predetermined length of time. According to the invention, secondary circuit 8 presents two filters hydraulically in series with each other, a first defined by a hemoperfusion cartridge 11 packed with uncoated activated carbon, and a second defined by a similar cartridge 12 packed with one (or more) of the aforementioned resins. Sampling points 15 are provided up- and downstream from filters 11, 12 to monitor purification of the blood in reservoir 4.

As shown in the Figure 2 to 7 graphs, and as described in more detail later on, the present invention provides for a rapid, steady reduction in the cytokines present in the blood in reservoir 4. A number of practical embodiments of the present invention will now be described by way of example.

## EXAMPLE 1

### - Characterization of adsorption on activated carbon and resins:

Uncoated activated carbon and the resins listed in Table 1 are packed, prewashed, by gravity into small columns (2.5 cm long by 1.2 cm inside diameter) formed from polystyrene tubes, to a total weight of 1.3 g. The resins are extensively washed and stored in a 50% solution of methanol and water. Immediately before perfusion, the cartridges so formed are washed with 20 times the volume of the cartridge (20 volumes) of sterile physiological saline solution and then conditioned with human plasma (10 ml). Normal human plasma is then prepared containing 2-2.5 $\mu$Ci (microcurie) of $^{125}$I labelled cytokines, such as TNF-$\alpha$ (NEN-Du Pont, specific activity 59.2 $\mu$Ci/mg), IL-1$\beta$ (Amersham, specific activity 3000 Ci/mmol), IL-6 (Amersham, specific activity 1100 Ci/mmol) and IL-8 (Amersham, specific activity 2000 Ci/mmol). Unlabelled cytokines are added to 100 ml of stirred plasma to the indicated concentration, and specifically 500 pg/ml for TNF-$\alpha$, 20 ng/ml for IL-1$\beta$, 50 ng/ml for IL-6, and 20 ng/ml for IL-8. After extensive stirring, 0.2 ml of plasma containing cytokines are counted in a $\gamma$-scintillation counter. Each cytokine is studied separately in a sterile single-pass circuit of the type in Figure 1, in which branch 8 is absent and filter 5 comprises the (resin or carbon) cartridge prepared as described above. The primary volume of the circuit used is 5 ml, and the blood flow rate through the test cartridge is maintained at 30 ml/hour. Aliquots of 0.2 ml are taken downstream from the test cartridge every 30 minutes. The amount of free iodine in the plasma samples is determined using the perchloric acid method and radioactivity counting in the supernatant. Perfusion is arrested when the radioactivity c.p.m. in the samples taken downstream from the cartridge equals that in the basal samples, thus indicating saturation of the cartridge. The cartridges are then disconnected from the circuit, washed in 20 volumes of isotonic saline solution, and opened. Aliquots of each tested adsorbent product are dried on filter paper, weighted and placed in tubes for counting. The results are expressed in ng of cytokine bound to 1 g of each adsorbent product, according to the following formula:

$$[(cpm\ bound/cpm\ basal)/mg\ of\ weighted\ sample]x100 \tag{1}$$

The results are shown in Table 1.

TABLE 1

| Adsorbent | TNF (ng/g) | IL-1 (ng/g) | IL-8 (ng/g) | IL-6 (ng/g) |
|---|---|---|---|---|
| Amberlite™ | 176 | 60 | 69 | 324 |
| Amberchrom™ | 395 | 235 | 725 | 168 |
| Ambersorb™ | 310 | 636 | 574 | 171 |
| Activ. carbon | 71 | 320 | 100 | 168 |

## EXAMPLE 2

### - Filtration/adsorption in an in vitro recirculating model

Four hundred (400) ml of fresh human blood drawn in ACD are added to 5 mg of LPS and incubated for 4 hours at 37°C. The blood is then recalcified, added to 5 U/ml of heparin (Liquemin, La Roche, Basle, Switzerland), and circulated in a closed-loop model as in Figure 1. An ultrafilter (BELLCO S.p.A. HFTO4 0.5 m$^2$ polysulfone membrane) is con-

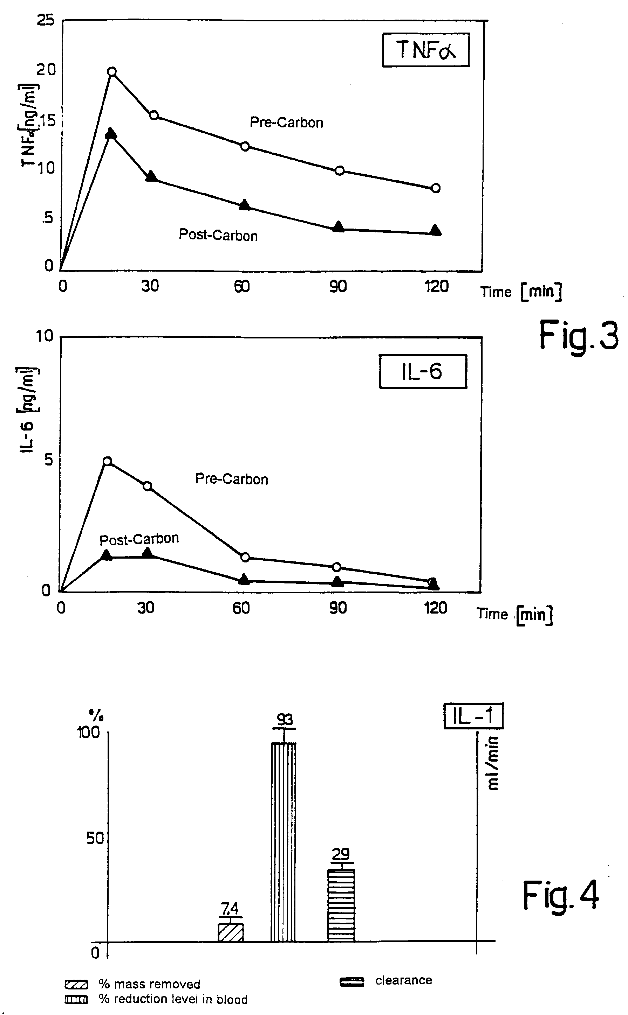

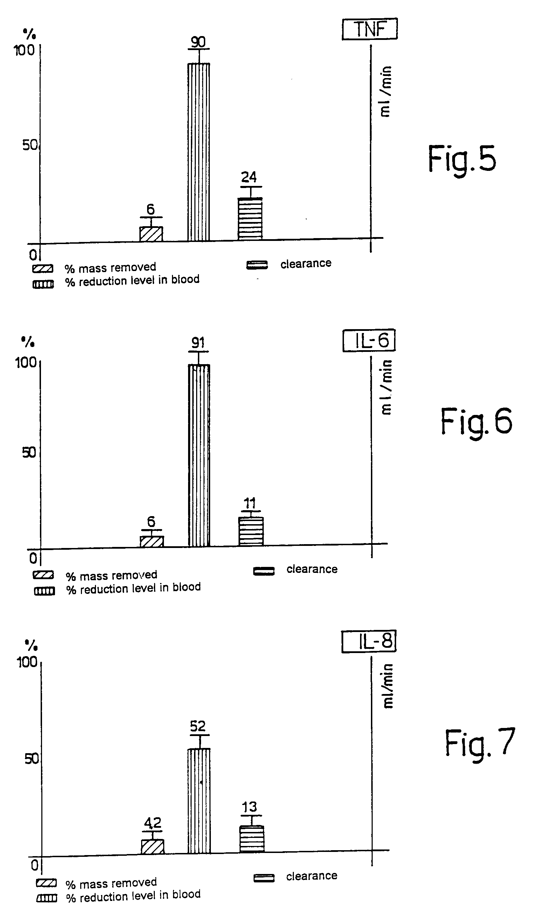

nected to the blood-side compartment of reservoir 4 containing the LPS-challenged blood. The ultrafiltrate is fed along branch 8 fitted with a single adsorbent cartridge 11 or 12 of 130 g of activated carbon or resin. After passing through the adsorbent cartridge, the ultrafiltrate is recirculated into reservoir 4. The blood flow rate in the main circuit is maintained at 200 ml/minute and the ultrafiltrate flow rate at 30 ml/minute by controlling the transmembrane pressure (TMP), and the duration of recirculation for each test is 120 minutes. Samples to determine IL-1$\beta$, TNF-$\alpha$, IL-8 and the receptor antagonist of IL-1 (IL-1ra) are taken upstream from the ultrafilter (as "blood levels") at different time intervals, i.e. after 0, 5, 15, 30, 60, 90 and 120 minutes; and further samples are taken up- and downstream from the adsorbent cartridge (points 15) at time intervals of 0, 15, 30, 60, 90 and 120 minutes. The blood samples are immediately centrifuged at 1000 rpm for 20 minutes at 4°C, and the recovered plasma is stored at -80°C until essayed to determine IL-1$\beta$, TNF-$\alpha$, IL-8 and the receptor antagonist of IL-1 (IL-1ra) using commercial ELISA kits. The results are shown in Figures 2 to 7. As can be seen, the resins not only provide for greater overall cytokine adsorption as compared with activated carbon (Table 1), but also for more rapidly eliminating high cytokine levels from the blood, as shown both by the high removal percentages and by the good clearance levels. As shown in Figures 2 and 3, however, activated carbon alone also provides for excellent cytokine removal from the ultrafiltrate, which is limited only towards TNF-$\alpha$, which, however, is largely retained (Figure 5) by the resins (90% elimination level and clearance of 24). Ideal application of the present invention therefore consists in double adsorption of the ultrafiltrate, first on carbon and then on resin, possibly selecting a specific resin (or combination of different resins) depending on the levels of the various cytokines to be absorbed, to ensure adequate working life of cartridges 11 and 12.

## Claims

1. A device for purifying blood comprising an infeed circuit (1) for a stream of blood to be purified; an ultrafilter or plasmafilter (5) in series with the infeed circuit (1); a collecting circuit (8) for collecting an ultrafiltrate or plasmafiltrate from said ultrafilter or plasmafilter (5), the collecting circuit (8) being branch connected in relation to the plasma-or ultrafilter (5); and filtering means (11, 12) hydraulically in series with the collecting circuit (8), said filtering means comprising a first adsorbent filter (11) packed with activated carbon and said plasmafilter or ultrafilter (5) being realized to allow passage into the ultrafiltrate or plasmafiltrate of at least any cytokines selected from the group consisting of: IL-1$\beta$, IL-6, IL-8, TNF-$\alpha$ and mixtures thereof;
   **characterized in that**, in combination with the above:

   - said filtering means further comprising a second adsorbent filter (12) packed with a hydrophobic or ion-exchange polystyrene resin, and located downstream from the first filter (11);

   - such that the device is able to be used for treating patients affected with acute organ failure.

2. A device as claimed in Claim 1, characterized in that the resin in the second filter (12) is a reticulate aromatic polymer, macroreticulate and/or macroporous resin and/or a synthetic carbonaceous resin, or any resin having performances equivalent to those of the known resins AMBERLITE™, AMBERCHROM™, AMBERSORB™.

3. A device as claimed in Claim 1 or 2, characterized in that said resins present a granule size of $2 \times 10^{-5}$ to $35 \times 10^{-5}$m, preferably of $1 \times 10^{-5}$ to $10 \times 10^{-5}$m or $25 \times 10^{-5}$ to $35 \times 10^{-5}$m (20 to 350 micron, preferably of 10 to 100 or 250 to 350 micron), and a porosity of $1 \times 10^{-8}$ - $3 \times 10^{-8}$m (100 to 300 Angstrom).

4. A device as claimed in Claim 1, characterized in that said hydrophobic resins are selected from the styrene-methacrylate and copolymer divinylbenzenepolystyrene group of resins.

5. A device as claimed in Claim 1, characterized in that said ion-exchange resins are selected from the synthetic carbonaceous group of resins.

## Patentansprüche

1. Eine Vorrichtung zum Reinigen von Blut umfassend einen Zuführkreislauf (1) für einen zu reinigenden Blutstrom; einen Ultrafilter oder Plasmafilter (5) in Reihe geschaltet mit dem Zuführkreislauf (1); einen Sammelkreislauf (8) zum Sammeln eines Ultrafiltrats oder Plasmafiltrats aus dem Ultrafilter oder Plasmafilter (5), wobei der Sammelkreislauf (8) in Bezug auf den Plasmafilter oder Ultrafilter (5) über eine Abzweigung verbunden ist; und Filtriermittel (11, 12), strömungstechnisch in Reihe geschaltet mit dem Sammelkreislauf (8), wobei die Filtriermittel einen ersten adsorbierenden Filter (11) umfassen, welcher mit Aktivkohle gepackt ist, und der Plasmafilter oder Ultrafilter (5) so verwirklicht ist, daß er den Durchgang in das Ultrafiltrat oder Plasmafiltrat von wenigstens irgendwelchen Zytokinen erlaubt, die ausgewählt sind aus der Gruppe bestehend aus: IL-1$\beta$, IL-6, IL-8, TNF-$\alpha$ und Mischungen davon; **dadurch gekennzeichnet, daß** in Kombination mit dem obigen:

- die Filtriermittel weiterhin einen zweiten adsorbierenden Filter (12) umfassen, der mit einem hydrophoben oder einem Ionenaustauscher-Polystyrol-Harz gepackt ist und stromabwärts von dem ersten Filter (11) angeordnet ist;

- so daß die Vorrichtung zur Behandlung von Patienten verwendet werden kann, die von einem akuten Organversagen betroffen sind.

2. Vorrichtung wie in Anspruch 1 beansprucht, dadurch gekennzeichnet, daß das Harz in dem zweiten Filter (12) ein retikuläres aromatisches Polymer, ein makroretikuläres und/oder makroporöses Harz und/oder ein synthetisches kohlenstoffhaltiges Harz oder irgendein Harz ist, welches eine Leistungsfähigkeit aufweist, die der von den bekannten Harzen AMBERLITE™, AMBERCHROM™, AMBERSORB™ entspricht.

3. Eine Vorrichtung wie in Anspruch 1 oder 2 beansprucht, dadurch gekennzeichnet, daß die Harze eine Körnchengröße von $2 \times 10^{-5}$ bis $35 \times 10^{-5}$ m, vorzugsweise $1 \times 10^{-5}$ bis $10 \times 10^{-5}$ m oder $25 \times 10^{-5}$ bis $35 \times 10^{-5}$ m (20 bis 350 Mikron, vorzugsweise 10 bis 100 oder 250 bis 350 Mikron) und eine Porosität von $1 \times 10^{-8}$ bis $3 \times 10^{-8}$ m (100 bis 300 Angström) aufweisen.

4. Vorrichtung wie in Anspruch 1 beansprucht, dadurch gekennzeichnet, daß die hydrophoben Harze ausgewählt sind aus der Gruppe der Styrol-Methacrylat- und der Copolymer-Divinylbenzol-Polystyrol-Harze.

5. Vorrichtung wie in Anspruch 1 beansprucht, dadurch gekennzeichnet, daß die Ionenaustauscherharze ausgewählt sind aus der Gruppe der synthetischen kohlenstoffhaltigen Harze.

**Revendications**

1. Dispositif pour purifier le sang comprenant un circuit (1) pour un prélévement de sang à purifier ; un ultrafiltre ou plasmafiltre (5) monté en série avec ledit circuit (1) ; un circuit de collecte (8) pour récupérer un ultrafiltrat ou plasmafiltrat provenant dudit ultrafiltre ou plasmafiltre (5), le circuit de collecte (8) étant branché en dérivation au plasmafiltre ou ultrafiltre (5) ; et des moyens de filtration (11, 12) hydrauliquement montés en série avec le circuit de collecte (8), lesdits moyens de filtration comprenant un premier filtre adsorbant (11) rempli de charbon actif et ledit plasmafiltre ou ultrafiltre (5) étant prévu pour permettre le passage dans l'ultrafiltrat ou le plasmafiltrat d'au moins quelques cytokines sélectionnées dans le groupe comprenant : IL-1$\beta$, IL-6, IL-8, TNF-$\alpha$ ou leurs mélanges ; caractérisé en ce que, en combinaison avec ce qui précède :

- lesdits moyens de filtration comprennent en plus un second filtre adsorbant (12) rempli d'une résine polystyrène hydrophobe ou échangeuse d'ions, et placé en amont du premier filtre (11);

- de sorte que le dispositif peut être utilisé pour traiter les patients atteints de déficiences d'organe aiguës.

2. Dispositif selon la revendication 1, caractérisé en ce que la résine dans le second filtre (12) est une résine de polymère aromatique réticulé, une résine macroréticulée et/ou macroporeuse et/ou une résine synthétique carbonée, ou les résines ayant des performances équivalentes à celles des résines connues AMBERLITE™, AMBERCHROM™, AMBERSORB™.

3. Dispositif selon la revendication 1 ou 2, caractérisé en ce que lesdites résines ont une granulométrie de $2 \times 10^{-5}$ à $35 \times 10^{-5}$m, de préférence de $1 \times 10^{-5}$ à $10 \times 10^{-5}$m ou de $25 \times 10^{-5}$ à $35 \times 10^{-5}$m (20 à 350 $\mu$m, de préférence de 10 à 100 ou de 250 à 350 $\mu$m), et une porosité de $1 \times 10^{-8}$ à $3 \times 10^{-8}$ m (100 à 300 angströms).

4. Dispositif selon la revendication 1, caractérisé en ce que lesdites résines hydrophobes sont choisies parmi les résines du groupe des styrène-méthacrylate et copolymère de divinylbenzène-polystyrène.

5. Dispositif selon la revendication 1, caractérisé en ce que lesdites résines échangeuses d'ions sont choisies parmi le groupe des résines synthétiques carbonées.

Fig.1

Fig.2

Fig.3

Fig.4

Fig.5

Fig.6

Fig. 7